# EUROPEAN PATENT APPLICATION

(11) **EP 4 775 144 A2**
(43) Date of publication of application: **15.07.2026**
(21) Application number: 26173714.2
(22) Date of filing: 23.06.2022
(51) Int. Cl.: A61B 5/15

(54) **CAPILLARY BLOOD COLLECTION DEVICE**

(30) Priority: 29.06.2021 US 202163216252 P
(62) Divisional of application: 22833938.8
(71) Applicant: Becton, Dickinson and Company, Franklin Lakes, NJ 07417 (US)
(72) Inventor: TORRIS, Anthony V., Montclair, 07042 (US); YAKHNICH, Vlad, Park Ridge, 07656 (US); WENTZELL, Scott, Suffern, 10901 (US); PORSCHEN, Leslie, Clifton, 07013 (US); FRICKE, Alex F., Bloomingdale, 07403 (US); BOKKA SRINIVASA RAO, Kishore K., 07450, Ridgewood (US); ALTHOFF, Charles Peter, West Nyack, 10994 (US)
(74) Representative: dompatent

(57) **Abstract**

A device for obtaining a blood sample may include a holder for receiving a sample source, the holder having an actuation portion and a port; a blood collector attachment removably connected to the holder; and a collection container removably connected to the blood collector attachment, the container defining a collection cavity, wherein a collection container detachment member is provided between the blood collector attachment and the collection container to releasably attach the collection container to the blood collector attachment.

## Description

### CROSS-REFERENCE TO RELATED APPLICATION

The present application aspects priority to United States Provisional Application Serial No. 63/216,252, filed June 29, 2021, entitled "Capillary Blood Collection Device", the entire disclosure of which is hereby incorporated by reference.

### BACKGROUND OF THE INVENTION

### Field of the Invention

The present disclosure relates generally to a device for obtaining a biological sample. More particularly, the present disclosure relates to an integrated finger-based capillary blood collection device with the ability to lance and squeeze a finger, collect, stabilize, and dispense a blood sample in a controlled manner.

### Description of Related Art

Devices for obtaining and collecting biological samples, such as blood samples, are commonly used in the medical industry. One type of blood collection that is commonly done in the medial field is capillary blood collection which is often done to collect blood samples for testing. Certain diseases, such as diabetes, require that the patient's blood be tested on a regular basis to monitor, for example, the patient's blood sugar levels. Additionally, test kits, such as cholesterol test kits, often require a blood sample for analysis. The blood collection procedure usually involves pricking a finger or other suitable body part in order to obtain the blood sample. Typically, the amount of blood needed for such tests is relatively small and a small puncture wound or incision normally provides a sufficient amount of blood for these tests. Various types of lancet devices have been developed which are used for puncturing the skin of a patient to obtain a capillary blood sample from the patient.

Many different types of lancet devices are commercially available to hospitals, clinics, doctors' offices, and the like, as well as to individual consumers. Such devices typically include a sharp-pointed member such as a needle, or a sharp-edged member such as a blade, that is used to make a quick puncture wound or incision in the patient's skin in order to provide a small outflow of blood. It is often physiologically and psychologically difficult for many people to prick their own finger with a hand-held needle or blade. As a result, lancet devices have evolved into automatic devices that puncture or cut the skin of the patient upon the actuation of a triggering mechanism. In some devices, the needle or blade is kept in a standby position until it is triggered by the user, who may be a medical professional in charge of drawing blood from the patient, or the patient himself or herself. Upon triggering, the needle or blade punctures or cuts the skin of the patient, for example, on the finger. Often, a spring is incorporated into the device to provide the "automatic" force necessary to puncture or cut the skin of the patient.

One type of contact activated lancet device that features automatic ejection and retraction of the puncturing or cutting element from and into the device is U.S. Patent No. 9,380,975, which is owned by Becton, Dickinson and Company, the assignee of the present application. This lancet device includes a housing and a lancet structure having a puncturing element. The lancet structure is disposed within the housing and adapted for movement between a retaining or pre-actuated position wherein the puncturing element is retained within the housing, and a puncturing position wherein the puncturing element extends through a forward end of the housing. The lancet device includes a drive spring disposed within the housing for biasing the lancet structure toward the puncturing position, and a retaining hub retaining the lancet structure in the retracted position against the bias of the drive spring. The retaining hub includes a pivotal lever in interference engagement with the lancet structure. An actuator within the housing pivots the lever, thereby moving the lancet structure toward the rearward end of the housing to at least partially compress the drive spring, and releases the lever from interference engagement with the lancet structure. The blood sample that is received is then collected and/or tested. This testing can be done by a Point-of-Care (POC) testing device or it can be collected and sent to a testing facility.

Currently, capillary blood collection workflow is a complex multi-step process requiring high skill level. The multi-step nature of this process introduces several variables that could cause sample quality issues such as hemolysis, inadequate sample stabilization, and micro-clots. The use of lancet devices for obtaining blood samples can result in several variables that effect the collection of the capillary blood sample, including, but not limited to, holding the lancet still during the testing, obtaining sufficient blood flow from the puncture site, adequately collecting the blood, preventing clotting, and the like. Some of the most common sources of process variability are: (1) inadequate lancing site cleaning and first drop removal which can potentially result in a contaminated sample; (2) inconsistent lancing location and depth which could potentially result in insufficient sample volume and a large fraction of interstitial fluid; (3) inconsistent squeezing technique and excessive pressure near the lancing site to promote blood extraction (e.g., blood milking) which could potentially result in a hemolyzed sample; (4) variable transfer interfaces and collection technique which could potentially result in a hemolyzed or contaminated sample; and (5) inadequate sample mixing with an anticoagulant which could potentially result in micro-clots.

Capillary collection blood draws are typically performed by health care workers either using their fingers to manually squeeze the tissue around the puncture site or by a device using vacuum pressure to pull blood from the site.

Manually squeezing the collection site is a highly technique dependent process that leads to very large variation in success rate and sample quality (as measured by hemolysis - blood cell rupture). Health care workers typically adjust the pressure and rate at which they squeeze to compensate for patient-dependent differences in blood flow. Squeezing harder helps blood flow more quickly but also increases hemolysis. The location of squeezing also varies between health care workers depending on personal preference, experience, and hand fatigue. Some workers may even perform a process called "milking" of fingers, where they apply pressure starting at the base of the finger and slide towards the tip of finger. This process is discouraged as leading to poor sample quality by domestic and international health organizations.

Vacuum-powered devices standardize the pressure and technique of blood flow, but are typically plagued by poor overall blood flow. The maximum pressure than can be applied is limited by the difference between atmospheric pressure and absolute vacuum (~14 psi), and devices only operate at a fraction of absolute vacuum. For reference, grip strength of men and women range from 50-100 lbs. on average, illustrating why manual methods are instead affected by hemolysis rather than flow. Vacuum methods also apply consistent pressure, limiting the ability of the tissue to replenish with blood.

Thus, there is a need in the art for a device that has the ability to lance and squeeze the finger, collect the sample, stabilize the sample, and subsequently dispense the sample in a controlled manner. There is also a need in the art for a device that simplifies and streamlines the capillary blood collection by eliminating workflow variabilities which are typically associated with low sample quality including hemolysis and micro-clots. There is still a further need in the art for a closed system collection and transfer that eliminate blood exposure and device reuse. There is still a further need in the art for a device that: (1) introduces flexibility in the accommodation of different capillary blood collection and transfer container; (2) has the capability to generate high quality uniformly mixed/stabilized capillary blood samples; (3) has the capability to generate on-board plasma from capillary plasma samples; (4) has the capability to collect large capillary blood samples (> 50-500µL) at reduced pain; (5) contains a unique sample identifier that is paired with patient information at the time of collection; (6) has the capability to collect capillary blood and perform on-board diagnostics; and (7) has multiple collection ports to collect a blood sample into different containers having the same or different anticoagulants. There is a further need in the art for a capillary blood collection device that includes a standardized and controlled location of applied pressure, an applied pressure that is high enough for adequate blood flow but below hemolysis thresholds, a defined rhythmic application of pressure rather than consistent pressure to allow blood to replenish in the finger, increasing average blood flow rate, and a reduced user fatigue by lowering maximum applied force by the operator.

### SUMMARY OF THE INVENTION

The present disclosure is directed to a device for obtaining a biological sample, such as a capillary blood collection device, which meets the needs set forth above and has the ability to lance and squeeze the finger, collect the sample, stabilize the sample, and subsequently dispense the sample in a controlled manner. The device also simplifies and streamlines the capillary blood collection by eliminating workflow variabilities which are typically associated with low sample quality including hemolysis and micro-clots.

The present disclosure includes a self-contained and fully integrated finger-based capillary blood collection device with ability to lance, collect, and stabilize high volume capillary blood sample, e.g., up to or above 500 microliters. The device simplifies and streamlines high volume capillary blood collection by eliminating workflow steps and variabilities which are typically associated with low sample quality including hemolysis, micro-clots, and patient discomfort. The device comprises a retractable lancing mechanism that can lance the finger and an associated blood flow path which ensures attachment and transfer of the capillary blood from the pricked finger site to the collection container. The device also includes a holder that can be cyclically squeezed to stimulate, i.e., pump, blood flow out of the finger and also an anticoagulant deposited in the flow path or collection container to stabilize collected sample.

According to one design, the device can comprise discrete components such as a holder, a lancet, and a collection container. According to another design, the lancet and collection container can be integrated into one device which is then used with the holder. According to yet another design, the holder, lancet, and collection container can be integrated into a single system. Any of these designs are envisioned to be used as a self-standing disposable device and/or in association with an external power source for pain reduction control. The capillary blood collection device can serve as a platform for various capillary blood collection containers ranging from small tubes to capillary dispensers, as well as on-board plasma separation modules. This capability extends the product flexibility to various applications including dispensing to a Point-of-Care (POC) cartridge or to a small collection tube transfer which can be used in a centrifuge or an analytical instrument.

In one embodiment of the present disclosure, a device for obtaining a blood sample may include a holder for receiving a sample source, the holder having an actuation portion and a port; a blood collector attachment removably connected to the holder; and a collection container removably connected to the blood collector attachment, the container defining a collection cavity, wherein a collection container detachment member is provided between the blood collector attachment and the collection container to releasably attach the collection container to the blood collector attachment.

In one embodiment of the present disclosure, the collection container may include a lid including a release tab that releases the collection container from the blood collector attachment when pressed. The lid may be connected to the collection container via a living hinge. The blood collector attachment may include a protrusion and the lid defines a recess to receive the protrusion to lock the collection container into the blood collector attachment. The living hinge may have a reduced thickness or at least one cutout. The lid may include at least one guide tab to ensure a desired orientation of the lid on the collection container when closing the lid. The collection container may include an aligning protrusion extending from an outer surface of the collection container and the blood collector attachment may define an aligning slot on an inner surface of the blood collector attachment to orient the collection container in a desired position when inserted into the blood collector attachment. A rotatable connection assembly may be provided between the holder and the blood collector attachment. The rotatable connection assembly may be configured to permit the collection container to rotate between a filling position and a stored position relative to the holder. The rotatable connection assembly may include a socket and a post member that are held together via a friction fit.

In one embodiment of the present disclosure, a device for obtaining a blood sample may include a blood collector attachment; and a collection container removably connected to the blood collector attachment, the container defining a collection cavity, wherein a collection container detachment member is provided between the blood collector attachment and the collection container to releasably attach the collection container to the blood collector attachment.

In one embodiment of the present disclosure, the collection container may include a lid including a release tab that releases the collection container from the blood collector attachment when pressed. The lid may be connected to the collection container via a living hinge. The blood collector attachment may include a protrusion and the lid defines a recess to receive the protrusion to lock the collection container into the blood collector attachment. The living hinge may have a reduced thickness or at least one cutout. The lid may include at least one guide tab to ensure a desired orientation of the lid on the collection container when closing the lid. The collection container may include an aligning protrusion extending from an outer surface of the collection container and the blood collector attachment may define an aligning slot on an inner surface of the blood collector attachment to orient the collection container in a desired position when inserted into the blood collector attachment. The blood collector attachment may define a relief recess to provide a location for a user's hand to grip when using the device. A recess may be defined in the blood collector attachment above the release tab to receive a user's thumb when using the device. When the collection container is held in the blood collector attachment, the lid may be held under pretension in the blood collector attachment.

The present invention is also described in the following clauses:
Clause 1: A device for obtaining a blood sample, the device comprising: a holder for receiving a sample source, the holder having an actuation portion and a port; a blood collector attachment removably connected to the holder; and a collection container removably connected to the blood collector attachment, the container defining a collection cavity, wherein a collection container detachment member is provided between the blood collector attachment and the collection container to releasably attach the collection container to the blood collector attachment.
Clause 2: The device of Clause 1, wherein the collection container comprises a lid including a release tab that releases the collection container from the blood collector attachment when pressed.
Clause 3: The device of Clause 2, wherein the lid comprises an elastomer that acts as a spring pre-loading a locking feature to create resistance with the collection container.
Clause 4: The device of any of Clauses 2-3, wherein the lid is connected to the collection container via a living hinge.
Clause 5: The device of any of Clauses 2-4, wherein the blood collector attachment comprises a protrusion and the lid defines a recess to receive the protrusion to lock the collection container into the blood collector attachment.
Clause 6: The device of any of Clause 3 or 4, wherein the living hinge has a reduced thickness or at least one cutout.
Clause 7: The device of any of Clauses 2-6, wherein the lid includes at least one guide tab to ensure a desired orientation of the lid on the collection container when closing the lid.
Clause 8: The device of any of Clauses 1-7, wherein the collection container comprises an aligning protrusion extending from an outer surface of the collection container and the blood collector attachment defines an aligning slot on an inner surface of the blood collector attachment to orient the collection container in a desired position when inserted into the blood collector attachment.
Clause 9: The device of any of Clauses 1-8, wherein a rotatable connection assembly is provided between the holder and the blood collector attachment.
Clause 10: The device of Clause 9, wherein, the rotatable connection assembly is configured to permit the collection container to rotate between a filling position and a stored position relative to the holder.
Clause 11: The device of any of Clauses 9-10, wherein the rotatable connection assembly includes a socket and a post member that are held together via a friction fit.
Clause 12: The device of Clauses 1-11, wherein the collection container detachment member provides haptic feedback when the collection container has been removed from the blood collector attachment.
Clause 13: A device for obtaining a blood sample, the device comprising: a blood collector attachment; and a collection container removably connected to the blood collector attachment, the container defining a collection cavity, wherein a collection container detachment member is provided between the blood collector attachment and the collection container to releasably attach the collection container to the blood collector attachment.
Clause 14: The device of Clause 13, wherein the collection container comprises a lid including a release tab that releases the collection container from the blood collector attachment when pressed.
Clause 15: The device of Clause 14, wherein the lid comprises an elastomer that acts as a spring pre-loading a locking feature to create resistance with the collection container.
Clause 16: The device of any of Clauses 14-15, wherein the lid is connected to the collection container via a living hinge.
Clause 17: The device of any of Clauses 13-16, wherein the blood collector attachment comprises a protrusion and the lid defines a recess to receive the protrusion to lock the collection container into the blood collector attachment.
Clause 18: The device of any of Clauses 13-17, wherein the living hinge has a reduced thickness or at least one cutout.
Clause 19. The device of any of Clauses 13-18, wherein the lid includes at least one guide tab to ensure a desired orientation of the lid on the collection container when closing the lid.
Clause 20: The device of any of Clauses 13-19 wherein the collection container comprises an aligning protrusion extending from an outer surface of the collection container and the blood collector attachment defines an aligning slot on an inner surface of the blood collector attachment to orient the collection container in a desired position when inserted into the blood collector attachment.
Clause 21: The device of any of Clauses 13-20, wherein the blood collector attachment defines a relief recess to provide a location for a user's hand to grip when using the device.
Clause 22: The device of any of Clauses 13-21, wherein a recess is defined in the blood collector attachment above the release tab to receive a user's thumb when using the device.
Clause 23: The device of any of Clauses 13-22, wherein, when the collection container is held in the blood collector attachment, the lid is held under pretension in the blood collector attachment.
Clause 24: The device of any of Clauses 13-23, wherein the collection container detachment member provides haptic feedback when the collection container has been removed from the blood collector attachment.

### BRIEF DESCRIPTION OF THE DRAWINGS

Fig. 1 is a perspective view of a holder in accordance with an embodiment of the present invention.
Fig. 2A is a cross-sectional view of a device for obtaining a blood sample from a patient's finger and a lancet in accordance with another embodiment of the present disclosure.
Fig. 2B is a perspective view of a device for obtaining a blood sample from a patient's finger and a sample collection container in accordance with another embodiment of the present disclosure.
Fig. 3 is a side view of a device for obtaining a blood sample from a patient's finger and a collection container in accordance with another embodiment of the present disclosure.
Fig. 4 is a perspective view of a collection container detachment member being unlocked.
Fig. 5 is a perspective view of the collection container of Fig. 4 in a detached state.
Fig. 6 is another perspective view of the collection container of Fig. 4 in the detached state.
Fig. 7 is a cross-sectional view of a blood collector attachment and collection container according to one embodiment of the present disclosure,
Fig. 8 is an enlarged view of the collection container of Fig. 7.
Fig. 9 is a perspective view of the collection container detachment member.
Fig. 10 is a perspective view of a collection container and a user's hand according to one embodiment of the present disclosure.
Fig. 11 is a perspective view of a device for obtaining a blood sample from a patient's finger and a collection container in accordance with another embodiment of the present disclosure.
Fig. 12 is a side view of the device of Fig. 11.
Fig. 13 is a front view of the device of Fig. 11.
Fig. 14 is a cross-sectional view of the collection container detachment member.
Fig. 15 is a side view of the collection container according to one embodiment of the present disclosure.
Fig. 16 is a top view of the collection container according to one embodiment of the present disclosure.
Fig. 17 is a top view of the collection container according to one embodiment of the present disclosure.
Fig. 18 is a perspective view the collection container with guide tabs according to one embodiment of the present disclosure.
Fig. 19 is a perspective view of the collection container and the blood collector attachment with aligning features according to one embodiment of the present disclosure.
Fig. 20 is a cross-sectional view of the collection container and the blood collector attachment with aligning features according to one embodiment of the present disclosure.
Fig. 21 is a side view of a rotatable connector assembly for a holder according to one embodiment of the present disclosure.
Fig. 22 is a cross-sectional view of the rotatable connector assembly of Fig. 21.
Fig. 23 is a cross-sectional view of an alternative rotatable connector assembly.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume alternative variations and step sequences, except where expressly specified to the contrary. It is also to be understood that the specific devices and processes illustrated in the attached drawings, and described in the following specification, are simply exemplary embodiments of the invention. Hence, specific dimensions and other physical characteristics related to the embodiments disclosed herein are not to be considered as limiting.

The present disclosure is directed to a device for obtaining a biological sample, such as a capillary blood collection device, which meets the needs set forth above and has the ability to lance and squeeze the finger, collect the sample, stabilize the sample, and subsequently dispense the sample in a controlled manner. The device also simplifies and streamlines the capillary blood collection by eliminating workflow variabilities which are typically associated with low sample quality including hemolysis and micro-clots.

Blood collection is fundamentally driven by pressure-driven flow. Devices or techniques either reduce the pressure outside the blood vessel (vacuum-powered flow) or increase the pressure inside the vessels. Both approaches increase the difference between the blood vessel pressure and external pressure, and increase the flow rate from inside the vessel to outside where the collection container is present. The location of squeezing can also be critical, as soft tissues (e.g. fat, skin, and musculature) are perfused with blood while hard tissues and joints are poorly perfused or are too mechanically stable to compress without patient pain.

Red blood cells (RBCs) are subject to hemolysis during collection. Hemolysis (RBC destruction) contaminates samples for diagnostic analysis, both by spilling cell contents into the liquid serum of the sample and by coloring the serum red via hemoglobin and interfering with colorimetric reactions. The amount of hemolysis during collection is driven by shear-mediated destruction of the cells due to flow rate and flow path as well as pressure-driven hemolysis where physical compression of tissues and vessels can damage cells. Hemolysis can therefore be controlled by ensuring that applied pressures and flows are not too high in any of the locations of the finger being squeezed.

The present disclosure includes a self-contained and fully integrated finger-based capillary blood collection device with ability to lance, collect, and stabilize high volume capillary blood sample, e.g., up to or above 500 microliters. The device simplifies and streamlines high volume capillary blood collection by eliminating workflow steps and variabilities which are typically associated with low sample quality including hemolysis, micro-clots, and patient discomfort. The device comprises a retractable lancing mechanism that can lance the finger and an associated blood flow path which ensures attachment and transfer of the capillary blood from the pricked finger site to the collection container. The device also includes a holder that can be cyclically squeezed to stimulate, i.e., pump, blood flow out of the finger and also an anticoagulant deposited in the flow path or collection container to stabilize collected sample.

According to one design, the device can comprise discrete components such as a holder, a lancet, and a collection container. According to another design, the lancet and collection container can be integrated into one device which is then used with the holder. According to yet another design, the holder, lancet, and collection container can be integrated into a single system. Any of these designs are envisioned to be used as a self-standing disposable device and/or in association with an external power source for pain reduction control. The capillary blood collection device can serve as a platform for various capillary blood collection containers ranging from small tubes to capillary dispensers, as well as on-board plasma separation modules. This capability extends the product flexibility to various applications including dispensing to a Point-of-Care (POC) cartridge or to a small collection tube transfer which can be used in a centrifuge or an analytical instrument.

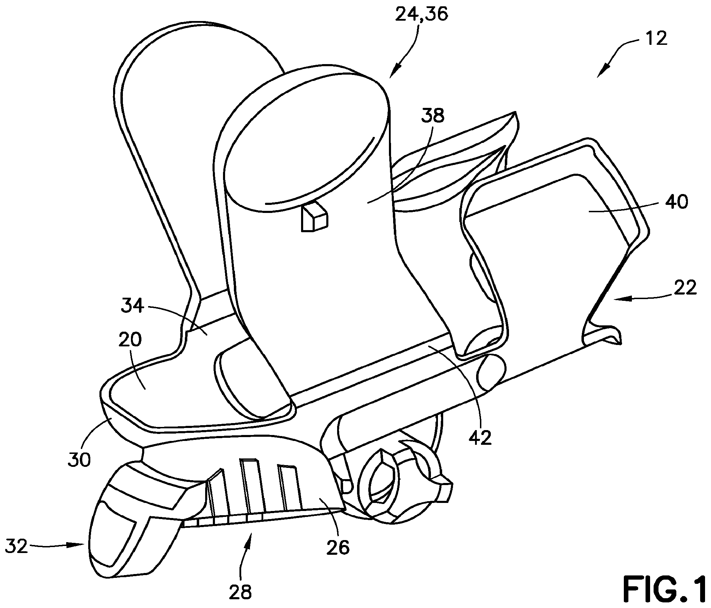

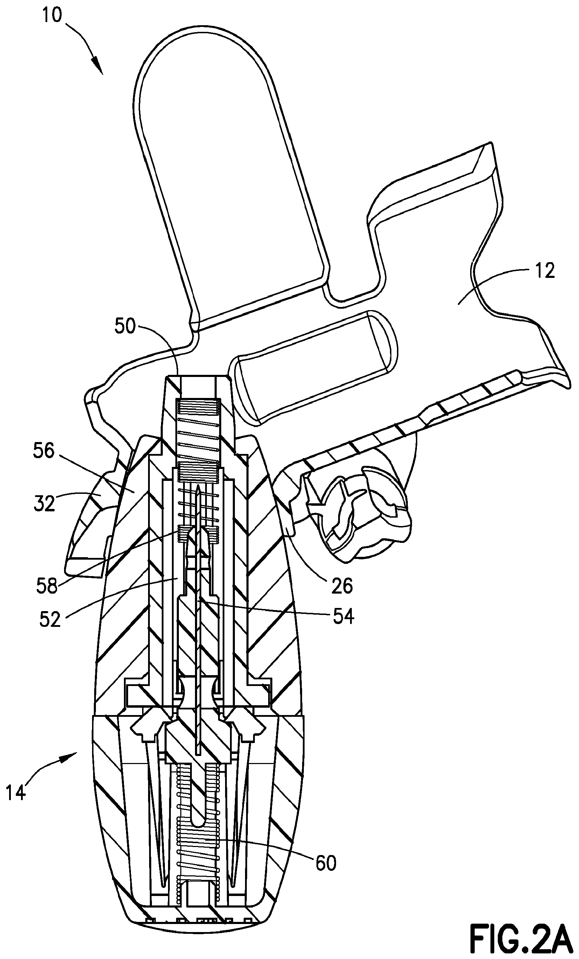

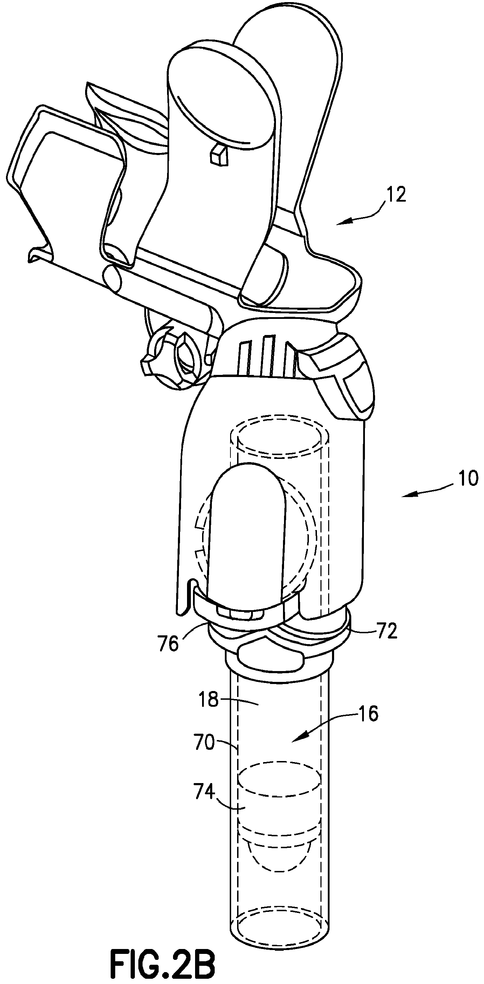

Referring to Figs. 1 and 2A, in an exemplary embodiment, a device 10 of the present disclosure includes discrete components, e.g., a holder 12 (as shown in Fig. 1), a lancet housing or lancet 14 (as shown in Fig. 2A), and a collection container 16 (as shown in Fig. 2B). In another exemplary embodiment, a semi-integrated device of the present disclosure may include an at-angle flow and include an integrated lancet housing and collection container which can be connected with a separate holder. In another exemplary embodiment, a semi-integrated device of the present disclosure may have an in-line flow and include an integrated lancet housing and collection container which can be connected with a separate holder. In another exemplary embodiment, an integrated device of the present disclosure may have an at-angle flow and include an integrated holder, lancet housing, and collection container. In another exemplary embodiment, an integrated device of the present disclosure may have an in-line flow and include an integrated holder, lancet housing, and collection container.

Referring to Fig. 1, an exemplary embodiment of a holder 12 of the present disclosure that is able to receive a sample source, e.g., a finger 19, for supplying a biological sample, such as a blood sample 18, is shown and described. A holder 12 of the present disclosure generally includes a finger receiving portion 20 having a first opening 22 (Fig. 1), an actuation portion 24, a port 26 having a second opening 28, and a finger end guard 30. In one embodiment, the finger end guard 30 provides a stop portion for properly aligning and securing a finger 19 within the holder 12. The finger end guard 30 further assists in ensuring the patient's finger 19 is placed at a proper position within the finger receiving portion 20 so that applied pressure to the patient's finger 19 will result in adequate blood flow.

The first opening 22 of the finger receiving portion 20 is configured for receiving a sample source, e.g., a finger 19, for supplying a biological sample, such as a blood sample 18. It can be appreciated that the sample source could include other parts of the body capable of fitting within the first opening 22. The port 26 is in communication with the finger receiving portion 20. For example, with a finger 19 received within the holder 12, the port 26 is in communication with a portion of the finger 19. A holder 12 of the present disclosure can be sized to accommodate all finger sizes.

The second opening 28 of the port 26 is configured for receiving a lancet housing 14 and a collection container 16 as described in more detail below. In one embodiment, the port 26 includes a locking portion 32 for securely receiving the lancet housing 14 and the collection container 16 within the port 26.

In one embodiment, the actuation portion 24 is transitionable between a first position in which the holder 12 defines a first diameter and a second position which the holder 12 defines a second diameter, wherein the second diameter is less than the first diameter. In one embodiment, the actuation portion 24 is transitionable between a first position in which the holder 12 defines a first elliptical shape, and a second position in which the holder 12 defines a second elliptical shape, wherein the first elliptical shape is different than the second elliptical shape. In this manner, with the holder 12 in the second position with a reduced diameter, a portion of the holder 12 contacts the sample source and the actuation portion 24 of the holder 12 is able to pump and/or extract blood 18 as described in more detail below.

Referring to Fig. 1, in one embodiment, the actuation portion 24 includes a contact member 34. With the actuation portion 24 in the first position, the contact member 34 is in a disengaged position, i.e., the contact member 34 is provided in a first position with respect to a sample source, e.g., the finger 19, such that the contact member 34 may be in slight contact therewith. With the actuation portion 24 in the second position, the contact member 34 is in an engaged position, i.e., the contact member 34 is provided in a second position with respect to the sample source, e.g., the finger 19, such that the contact member 34 is in an applied pressure contact with the finger 19, and the actuation portion 24 of the holder 12 is able to pump and/or extract blood 18. For example, with the contact member 34 in the engaged position, the contact member 34 exerts a pressure on the sample source.

Referring to Fig. 1, in one embodiment, the actuation portion 24 includes a pumping member 36 for applying pressure to the sample source, e.g., the finger 19. In one embodiment, the pumping member 36 comprises a pair of opposed tabs or wings 38. In such an embodiment, each tab 38 may include a contact member 34. In one embodiment, the holder 12 includes a living hinge portion 42. The living hinge portion 42 allows a user to squeeze the wings 38 between a first position (passive state) and a second position (active state). The use of the tabs or wings 38 to draw blood 18 out of a patient's finger 19 minimizes hemolysis while maintaining an adequate flow of blood from the patient's finger 19. A resting position and hinge of the wings 38 are designed to maintain contact and retention with the smallest patient finger that can fit into a holder 12 while flexing to accommodate the largest patient finger within a holder 12 without blood occlusion.

Advantageously, the holder 12 of the present disclosure allows a user to repeatedly squeeze and release the wings 38 to pump and/or extract blood 18 from a finger 19 until a desired amount of blood 18 is filled in a collection container 16. The wings 38 are configured to flex to maintain gentle contact with a range of patient finger sizes that may be used with the holder 12 and to retain the holder 12 on the patient's finger 19.

Advantageously, with the holder 12 placed onto a finger 19, the holder 12 does not constrict the blood flow and defines lancing and finger squeezing locations. The squeezing tabs or wings 38 provide a pre-defined range of squeezing pressure that is consistently applied throughout a finger 19. By doing so, the holder 12 provides a gentle controlled finger massage that stimulates blood extraction and minimizes any potential hemolysis.

Referring to Fig. 1, in one embodiment, the holder 12 includes a stability extension portion 40. This provides additional support for the holder 12 to be securely placed onto a finger 19. In one embodiment, the finger receiving portion 20 forms a generally C-shaped member and includes a plurality of inner gripping members for providing additional grip and support for the holder 12 to be securely placed onto a finger 19. The stability extension portion 40 assists in maintaining contact with the patient's finger 19 during use of the holder 12 while avoiding the blood supply and knuckles of the patient's finger 19.

In one embodiment, the finger receiving portion 20 is formed of a flexible material. In some embodiments, the finger receiving portion 20 and the port 26 are formed from a flexible material.

A device 10 for obtaining a blood sample 18 of the present disclosure includes a lancet housing or lancet 14 that is removably connectable to a port 26 of a holder 12. In one embodiment, the lancet housing 14 includes an inlet or opening 50, an interior 52, a puncturing element 54, an engagement portion 56, a retractable mechanism 58, and a drive spring 60. In one embodiment, the puncturing element 54 is moveable between a pre-actuated position wherein the puncturing element 54 is retained within the interior 52 of the lancet housing 14 and a puncturing position wherein at least a portion of the puncturing element 54 extends through the inlet 50 of the lancet housing 14 to lance a portion of a finger 19.

In one embodiment, the lancet 14 of the present disclosure is a contact activated lancet and may be constructed in accordance with the features disclosed in U.S. Patent Application Publication No. 2006/0052809 filed May 6, 2005, entitled "Contact Activated Lancet Device", and commonly assigned with the present application, the entire disclosure of which is hereby expressly incorporated herein by reference thereto.

In one embodiment, the lancet housing 14 may be a separate component from the holder 12 and the collection container 16. In some embodiments, the collection container 16 and the lancet housing 14 form a single component that is removably connectable to the port 26 of the holder 12. In some embodiments, the collection container 16, the lancet housing 14, and the holder 12 form a single component.

Referring to Fig. 2A, in one embodiment, with the holder 12 and the lancet housing 14 being separate components, the lancet housing 14 is removably connectable to the port 26 of the holder 12. In such an embodiment, the lancet housing 14 includes an engagement portion 56. Referring to Fig. 2A, in one embodiment, the lancet housing 14 is pushed into the port 26 of the holder 12 such that the engagement portion 56 of the lancet housing 14 is locked within the locking portion 32 of the holder 12. In this manner, the lancet housing 14 is securely connected and locked to the holder 12 such that the puncturing element 54 of the lancet housing 14 can be activated to lance or puncture a sample source, e.g., a finger 19. In some embodiments, the port 26 of the holder 12 includes a plurality of ribs for securing and locking the lancet 14 or the collection container 16 in the port 26.

To activate the lancet 14, the lancet 14 is pushed against a finger 19 to activate a retractable mechanism 58 of the lancet 14 to lance a finger 19. The lancet 14 of the present disclosure consistently delivers correct lancing depth and a pre-defined lancing location, thus ensuring a sufficient sample volume.

In one embodiment, the lancet 14 includes a drive spring 60 disposed within the interior 52 of the lancet housing 14 for biasing the puncturing element 54 toward the puncturing position. After puncturing, the puncturing element 54 is immediately retracted and safely secured within the interior 52 of the lancet housing 14.

In one embodiment, the lancet 14 of the present disclosure is used to lance the skin of a finger 19 and then a blood sample 18 is squeezed into a collection container 16 as described in more detail below.

In one embodiment, the lancet housing 14 of the present disclosure is used to lance the skin of a finger 19 along a lance path and then a blood sample 18 flows down a blood flow path at an angle to the lance path as described in more detail below.

In one embodiment, the lancet 14 can include a hollow needle. In such an embodiment, the lancet housing 14 of the present disclosure is used to lance the skin of a finger 19 along a lance path and then a blood sample 18 (shown in Fig. 2B) flows along a parallel blood flow path through the hollow needle.

As shown in Fig. 2B, a device 10 for obtaining a blood sample 18 of the present disclosure includes a collection container 16 that is removably connectable to the port 26 of the holder 12. The collection container 16 defines a collection cavity 70 for receiving a blood sample 18, a container engagement portion 72, a blood collector portion 74, and a cap or septum 76. Once a desired amount of blood 18 is collected within the container 16, a blood collector portion 74 is detached from the collection device 10 in order to send a collected sample 18 to a diagnostic instrument and/or testing device. The blood collector portion 74 is sealed via the cap or septum 76 once removed from the collection device 10 to protectively seal the blood sample 18 within the collection cavity 70.

In one embodiment, the collection container 16 may be a separate component from the holder 12 and the lancet housing 14. In some embodiments, the collection container 16 and the lancet housing 14 form a single component that is removably connectable to the port 26 of the holder 12. In some embodiments, the collection container 16, the lancet housing 14, and the holder 12 form a single component.

In one embodiment, with the holder 12 and the collection container 16 being separate components, the container 16 is removably connectable to the port 26 of the holder 12. In such an embodiment, the container 16 includes a container engagement portion 72. In one embodiment, the container 16 is pushed into the port 26 of the holder 12 such that the container engagement portion 72 of the container 16 is locked within the locking portion 32 of the holder 12. In this manner, the container 16 is securely connected and locked to the holder 12 such that a blood sample 18 can safely flow from the finger 19 within the holder 12 to the collection cavity 70 of the container 16.

It can be appreciated that several types of collection containers 16 can be used with the device 10 of the present disclosure. It can also be appreciated that the collection container 16 can be associated with a separate dispensing unit or the collection container 16 can include an integral dispensing portion for dispensing the blood 18 to a testing device.

Referring to Fig. 1, use of a device 10 of the present disclosure having discrete components, e.g., a holder 12, a lancet housing or lancet 14, and a collection container 16, will now be described.

Referring to Fig. 1, first a desired finger 19 is cleaned and a holder 12 having an appropriate size for the desired finger 19 is selected and placed onto the finger 19 securely. Next, referring to Fig. 2A, a lancet housing 14 is connected to the port 26 of the holder 12. As discussed above, the lancet housing 14 is pushed into the port 26 of the holder 12 such that the engagement portion 56 of the lancet housing 14 is locked within the locking portion 32 of the holder 12. In this manner, the lancet housing 14 is securely connected and locked to the holder 12 such that the puncturing element 54 (Fig. 2A) of the lancet housing 14 can be activated to lance or puncture a sample source, e.g., a finger 19. With the lancet 14 connected to the port 26 of the holder 12, the lancet 14 is in communication with the finger 19.

When it is desired to activate the lancet 14 to lance the skin of a finger 19, the lancet 14 is pushed against a finger 19 to activate a retractable mechanism 58 (Fig. 2A) of the lancet 14 to lance a finger 19. The lancet 14 of the present disclosure consistently delivers correct lancing depth and a pre-defined lancing location, thus ensuring a sufficient sample volume.

After the finger 19 is lanced to create blood 18 flow from the finger 19, the lancet 14 is removed from the holder 12 and the collection container 16 is pushed into the port 26 of the holder 12. Referring to Fig. 2B, the container 16 is pushed into the port 26 of the holder 12 such that the container engagement portion 72 of the container 16 is locked within the locking portion 32 of the holder 12. In this manner, the container 16 is securely connected and locked to the holder 12 such that a blood sample 18 can safely flow from the finger 19 within the holder 12 to the collection cavity 70 of the container 16.

Referring to Fig. 1, with the container 16 properly secured to the holder 12 for collection of a blood sample 18, a user is able to repeatedly squeeze and release the wings 38 of the holder 12 to pump and/or extract blood 18 from a finger 19 until a desired amount of blood 18 is filled in a collection container 16. Advantageously, with the holder 12 placed onto a finger 19, the holder 12 does not constrict the blood flow and defines lancing and finger squeezing locations. The squeezing tabs or wings 38 provide a pre-defined range of squeezing pressure that is consistently applied throughout a finger 19. By doing so, the holder 12 provides a gentle controlled finger 19 massage that stimulates blood extraction and minimizes any potential hemolysis.

For example, referring to Fig. 1, in one embodiment, the actuation portion 24 includes a contact member 34. With the actuation portion 24 in the first position, the contact member 34 is in a disengaged position, i.e., the contact member 34 is in the first position with respect to the sample source, e.g., the finger 19. With the actuation portion 24 in the second position, the contact member 34 is in an engaged position, i.e., the contact member 34 is in the second position and in applied pressure contact with a sample source, e.g., the finger 19, and the actuation portion 24 of the holder 12 is able to pump and/or extract blood 18. For example, with the contact member 34 in the engaged position, the contact member 34 exerts a pressure on the sample source.

Once a desired amount of blood 18 is collected within the container 16, a blood collector portion 74 is detached from the collection device 10 in order to send a collected sample 18 to a diagnostic instrument and/or testing device. The blood collector portion 74 is sealed via the cap or septum 76 once removed from the collection device 10 to protectively seal the blood sample 18 within the collection cavity 70.

The devices of the present disclosure are compatible with any known testing device, whether the testing device is off-site or a point-of-care testing device. Various point-of-care testing devices are known in the art. Such point-of-care testing devices include test strips, glass slides, diagnostic cartridges, or other testing devices for testing and analysis. Test strips, glass slides, and diagnostic cartridges are point-of-care testing devices that receive a blood sample and test that blood for one or more physiological and biochemical states. There are many point-of-care devices that use cartridge based architecture to analyze very small amounts of blood bedside without the need to send the sample to a lab for analysis. This saves time in getting results over the long run, but creates a different set of challenges versus the highly routine lab environment. Examples of such testing cartridges include the i-STAT^{®} testing cartridge from the Abbot group of companies. Testing cartridges such as the i-STAT^{®} cartridges may be used to test for a variety of conditions including the presence of chemicals and electrolytes, hematology, blood gas concentrations, coagulation, or cardiac markers. The results of tests using such cartridges are quickly provided to the clinician.

The collection container 16 may also contain a sample stabilizer, e.g., an anticoagulant, to stabilize a blood sample 18 and/or a component of a blood sample 18 disposed therein. The collection container 16 may also include at least one fill line(s) corresponding to a predetermined volume of sample. The collection container may also indicate/meter a collected volume of blood.

Any of the devices for obtaining a blood sample of the present disclosure can be used as a self-standing disposable device and/or in association with an external power source for pain reduction control. For example, a portion of holder 12 may include embedded electrodes which receive a signal from an external pain control module to deliver at least one of heat, vibration, or transcutaneous electrical nerve stimulation (TENS) for pain reduction control. The devices for obtaining a blood sample of the present disclosure may also include various options for on-board plasma separation. The devices for obtaining a blood sample of the present disclosure may also include a unique sample identifier that can be paired with patient information at the time of collection. The devices for obtaining a blood sample of the present disclosure may also include on-board diagnostic feedback at the time of collection. A device for obtaining a blood sample of the present disclosure may also allow for dual collection, e.g., the collection of two samples into two separate containers, using multiple collection ports which enable the collection of multiple samples from the same source and treating the samples with different sample stabilizers, such as anticoagulants.

A device for obtaining a blood sample of the present disclosure significantly simplifies and de-skills large volume capillary collection from a finger relative to the conventional capillary collection using lancet and capillary tube. The devices of the present disclosure eliminate blood exposure and prevents device reuse.

The devices for obtaining a blood sample of the present disclosure simplify, deskill, and streamline the collection process. This is all achieved by a self-contained closed system device which after it is placed onto a finger will provide lancing, blood extraction, stabilization, and containment functions, all in one unit.

The devices for obtaining a blood sample of the present disclosure may be associated with a self-standing unit that provides automated pumping, controlled finger squeezing, and automated sample labeling and processing.

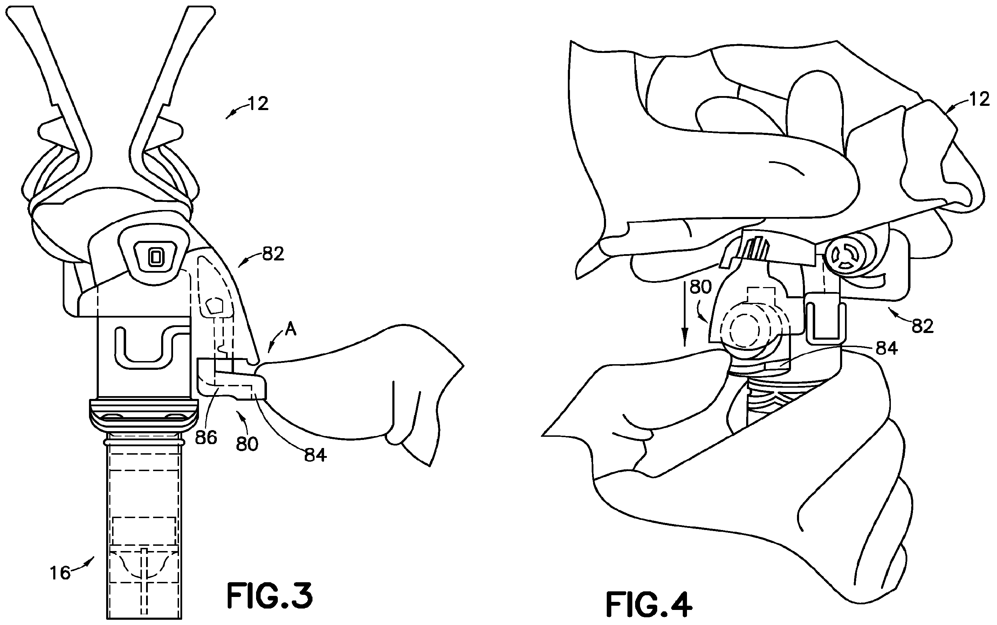

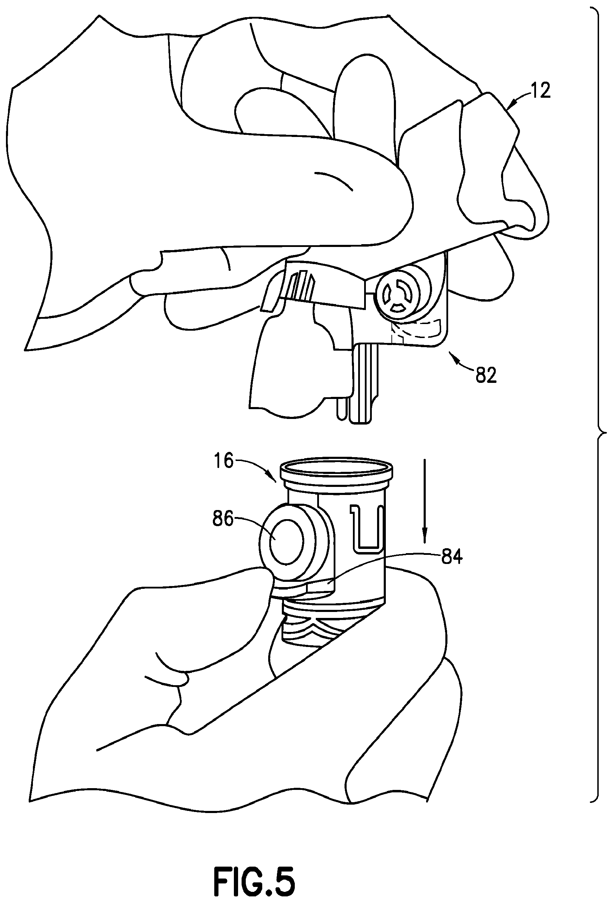

With reference to Figs. 3-5, according to one embodiment of the present disclosure, a collection container detachment member 80 is shown and described in detail. The collection container detachment member 80 may be an intuitive release mechanism that permits a user to press release the member 80 to release the collection container 16 from a blood collector attachment 82 removably connected to the holder 12. The collection container detachment member 80 provides haptic feedback to the user to indicate that the collection container 16 has been removed from the blood collector attachment 82.

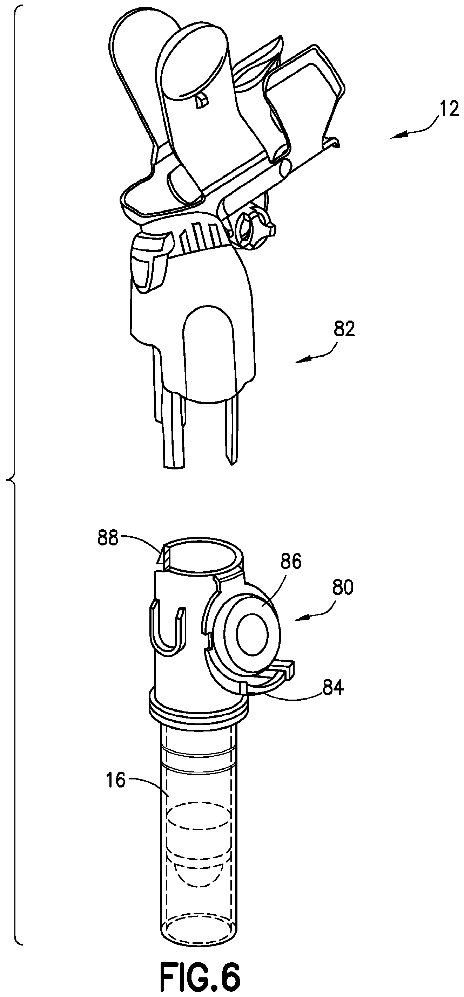

With reference to Fig. 6, according to one embodiment of the present disclosure, the collection container detachment member 80 may include a release tab 84 that permits a user to effect a disconnection of the collection container 16 from the blood collector attachment 82. In one embodiment, the release tab 84 may be pressed inwardly by the user to release the collection container 16 from the blood collector attachment 82 after the blood sample 18 has been drawn from the patient. Once the release tab 84 has been pressed by the user, the collection container 16 may be pulled away from the blood collector attachment 82. The release tab 84 may include a latch feature that prevents contamination to the surfaces that contact the blood sample 18 in the collection container 16. Since the release tab 84 must be positively engaged by the user to release the collection container 16, the release tab 84 prevents any accidental release of the collection container 16 from the blood collector attachment 82. In one embodiment, the release tab 84 may have a bright color or pattern that visually identifies to the user that the release tab 84 must be pressed to release the collection container 16 from the blood collector attachment 82. In one embodiment, the release tab 84 acts as a "lever" for unlocking the collection container detachment member 80. In one embodiment of the present disclosure, the blood collector attachment 82 may also shield the sealing surface 106 of the lid 86 from contamination until the collection container 16 is removed and the lid 86 is closed on the collection container 16. In one embodiment of the present disclosure, the latching feature of the lid 86 may be blocked by the blood collector attachment 82 to provide a tamper-proof feature that identifies tampering of the lid 86.

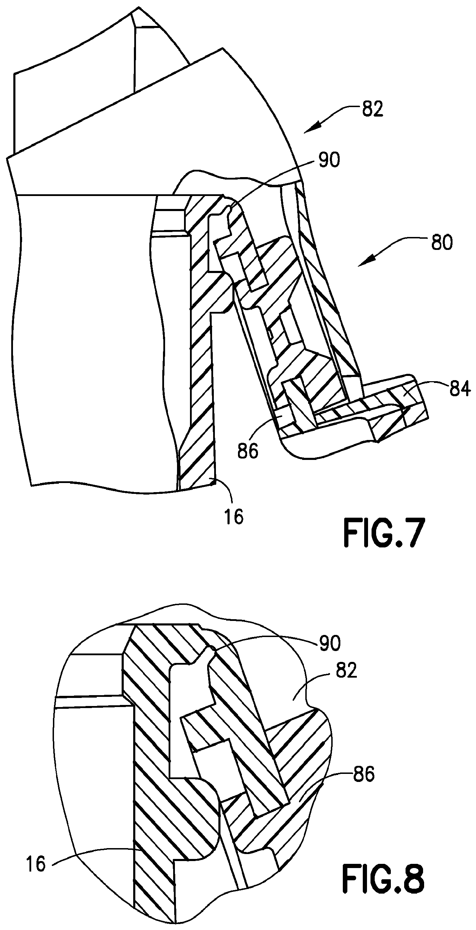

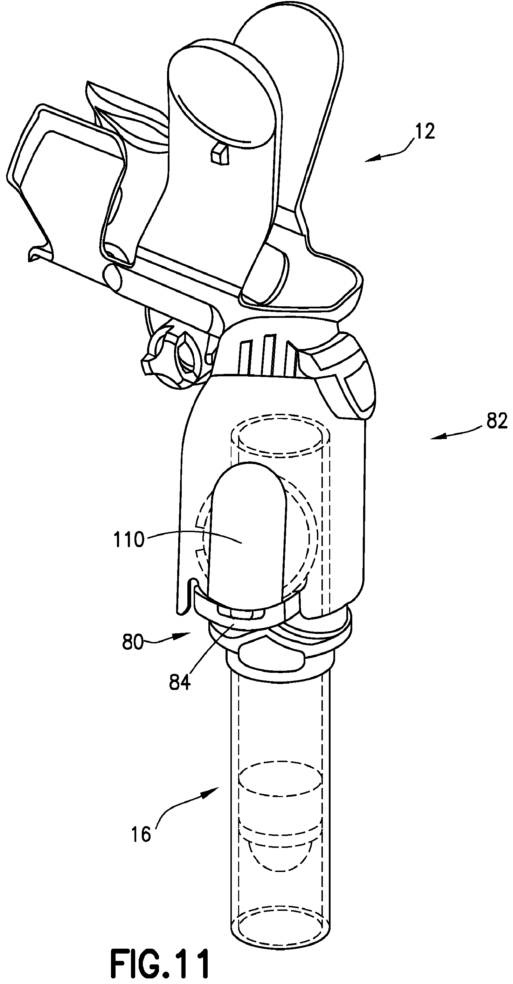

As shown in Figs. 7 and 9-13, according to one embodiment of the present disclosure, a lid 86 of the collection container 16 may rest against an outer protrusion 88 that extends outwardly from an outer surface of the collection container 16. The lid 86 may have a soft rubber like material (TPE) that acts as a spring pre-loading the locking features and creates resistance to the user pressing it while detaching the collection container 16. The release tab 84 may be formed as a protrusion on the lid 86. The lid 86 may be connected to the collection container 16 using a living hinge 90. The lid 86 may be preloaded with a force that causes the lid 86 to slightly deflect. In one embodiment, the lid 86 may be under a 50%-75% preload. When the lid 86 is provided under the preload, the living hinge 90 may act as a spring, as shown in Fig. 8. An inner portion of the living hinge 90 may experience compression, while the outer portion of the living hinge 90 may experience tension. The preload on the lid 86 assists in retaining the collection container 16 in a positive locking engagement with the blood collector attachment 82.

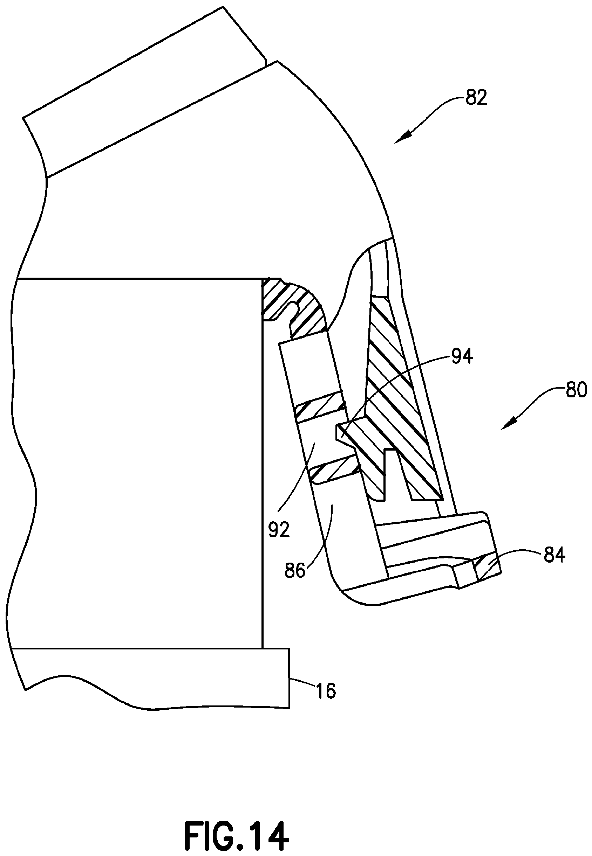

With reference to Figs. 3 and 14, as the user pushes against a lip of the lid 86 in a direction A, the lid 86 may experience further deflection. The lid 86 may be configured so as to stand out or away from the collection container 16 even after the user pushes the release tab 84 towards the collection container 16. As shown in Fig. 14, in one embodiment of the present disclosure, the lid 86 may include a recess 92 that is configured to engage with a protrusion 94 extending from the blood collector attachment 82. When the collection container 16 is inserted into the blood collector attachment 82, the lid 86 is pressed against the protrusion 94 until the protrusion 94 positively locks into the recess 92 formed on the lid 86 of the collection container 16. Corresponding surfaces on the recess 92 and the protrusion 94 are configured to positively engage one another to retain the lid 86 in the open position until released by the release tab 84. Once a first collection container 16 is filled with a blood sample 18, the first collection container 16 may be easily removed from the blood collector attachment 82 and a second collection container 16 may be inserted into the blood collector attachment 82 to receive an additional volume of the blood sample 18.

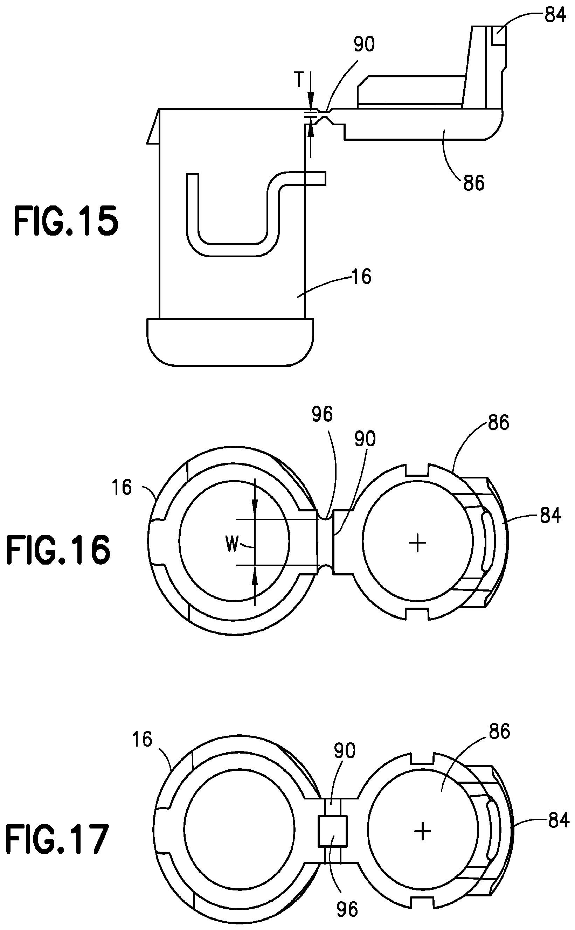

With reference to Figs. 15-17, according to various embodiments of the present disclosure, the bending line and stiffness of the living hinge 90 may be adjusted by adding or removing material from the living hinge 90. In Fig. 15, the thickness T of the living hinge 90 may be decreased to adjust the bending line and stiffness of the living hinge 90. In Fig. 16, cutouts or slots 96 may be made in the living hinge 90 to reduce the width W of the living hinge 90. In Fig. 17, a cutout or slot 96 may be cut into the living hinge 90 to decrease the amount of material used in the living hinge 90. It is to be understood that other portions of the living hinge 90 may have material removed or added to control the bending line and stiffness of the living hinge 90.

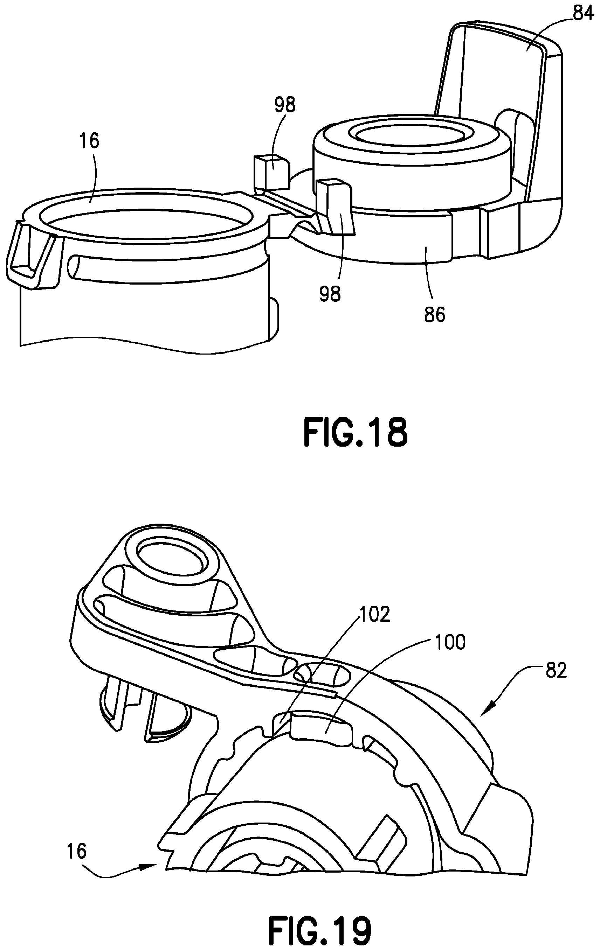

With reference to Fig. 18, in one embodiment of the present disclosure, the lid 86 of the collection container 16 may include at least one guide tab 98 to ensure the lid 86 is accurately positioned on the collection container 16 when the lid 86 is shut. In one embodiment of the present disclosure, the collection container 16 may include two guide tabs 98, with one guide tab 98 positioned on each side of the lid 86 to engage the sides of the collection container 16.

With reference to Fig. 19, in one embodiment of the present disclosure, the collection container 16 and the blood collector attachment 82 may include aligning features to ensure the collection container 16 is properly aligned when received in the blood collector attachment 82. In one embodiment, the collection container 16 may include an aligning protrusion 100 protruding from an outer surface of the collection container 16. A corresponding aligning slot 102 may be defined in an inner surface of the blood collector attachment 82 to receive the aligning protrusion 100. It is also contemplated that the blood collector attachment 82 may include the aligning protrusion 100 and the collection container 16 may include the aligning slot 102. In some embodiments of the present disclosure, the collection container 16 may include more than one aligning protrusion 100, while the blood collector attachment 82 includes more than one aligning slot 102. In one embodiment, the number of aligning protrusions 100 provided on the collection container 16 is the same as the number of aligning slots 102 provided on the blood collector attachment 82.

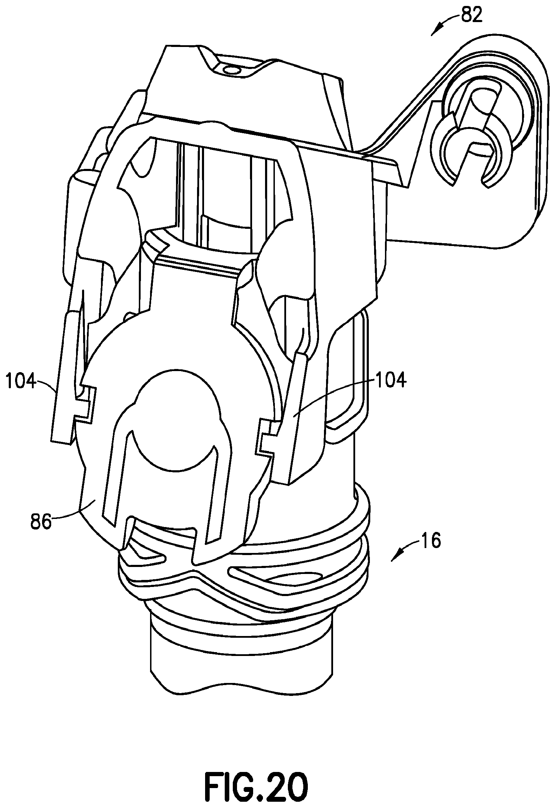

With reference to Fig. 20, in one embodiment of the present disclosure, the blood collector attachment 82 may include additional features for guiding the collection container 16 into the desired orientation when being inserted into the blood collector attachment 82. Side walls 104 of the blood collector attachment 82 are configured as ramps to guide the collection container 16 into a positive engagement with the blood collector attachment 82 to hold the collection container 16 in secure placement in the blood collector attachment 82.

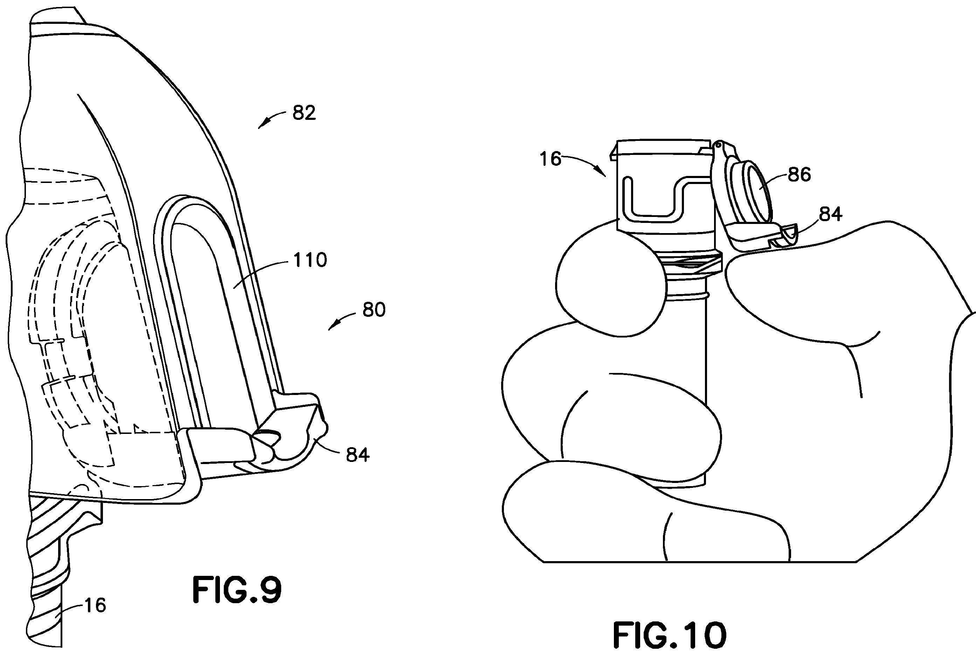

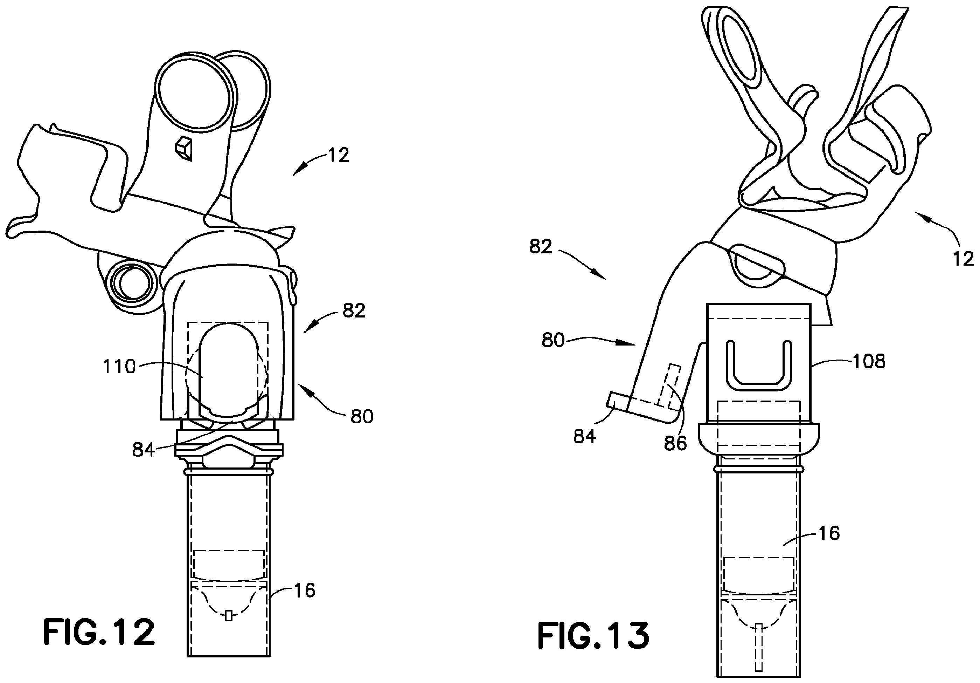

As shown in Fig. 13, in one embodiment of the present disclosure, a relief recess 108 may be defined in an outer surface of the blood collector attachment 82 to assist in improving a user's grip on the blood collector attachment 82. The relief recess 108 may be positioned opposite the release tab 84 that is pressed by the user's thumb. With reference to Fig. 9, in one embodiment of the present invention, a concave recess 110 may be defined in the outer surface of the blood collector attachment 82 at a location above the release tab 84. The recess 110 may be provided to bring the release tab 84 touchpoints further from the surfaces of the blood collector attachment 82. The recess 110 also provides a visual "landing pad" for the user's thumb when operating the blood collector attachment 82. The concave surfaces of the recess 110 may also be heavily textured to mute the background features of the blood collector attachment 82.

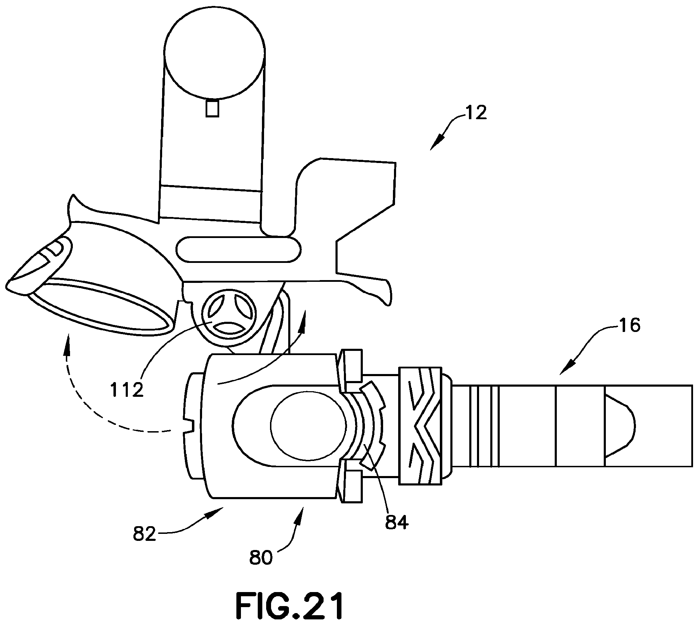

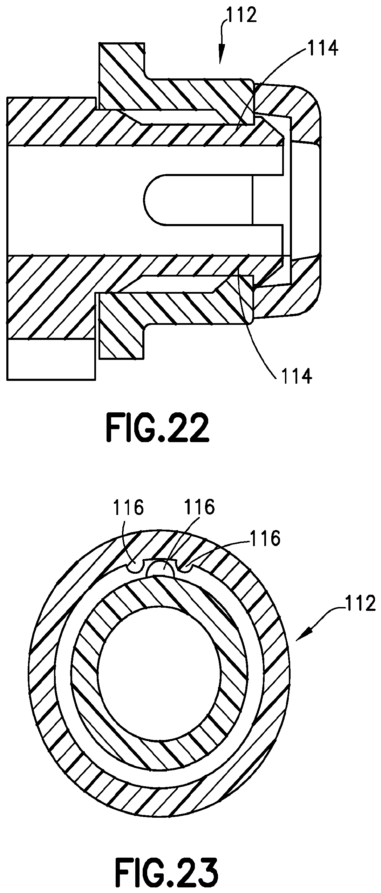

With reference to Figs. 21 and 22, in one embodiment of the present disclosure, the holder 12 may include a rotatable connection assembly 112 connected to the blood collector attachment 82 to permit the user to rotate the blood collector attachment 82 and the collection container 16 away from and towards the port 26 on the holder 12. The rotatable connection assembly 112 may include a socket and post member that are positively engaged via a friction fit to permit rotation of the blood collector attachment 82 and the collection container 16. The socket and post member may be provided on either the holder 12 or the blood collector attachment 82, respectively. The friction fit for the rotatable connection assembly 112 is strong enough to support the movement force created by the collection container 16 when held in the inactive position to keep the collection container 16 from interfering with the lancet 14 when puncturing the patient's finger 19. Further, the friction fit for the rotatable connection assembly 112 is strong enough to require a user to manually rotate the collection container 16 into the active state. The collection container 16 is unable to swing into the active state on under its own weight.

As shown in Fig. 22, in one embodiment of the present disclosure, the rotatable connection assembly 112 includes friction bearing surfaces 114 that create the friction fit to retain the collection container 16 in the inactive state. In one embodiment, the coefficient of friction between the friction bearing surfaces 114 is 0.15-0.2. As shown in Fig. 23, in another embodiment of the present disclosure, the rotatable connection assembly 112 may include retaining bumps 116 that prevent rotation of the rotatable connection assembly 112 until a sufficient force is applied.

While an embodiment of a capillary blood collection device is shown in the accompanying figures and described hereinabove in detail, other embodiments will be apparent to, and readily made by, those skilled in the art without departing from the scope and spirit of the invention. Accordingly, the foregoing description is intended to be illustrative rather than restrictive. The invention described hereinabove is defined by the appended aspects and all changes to the invention that fall within the meaning and the range of equivalency of the aspects are to be embraced within their scope.

### FURTHER ASPECTS

[Aspect 1] A device for obtaining a blood sample, the device comprising:
   a holder for receiving a sample source, the holder having an actuation portion and a port;
   a blood collector attachment removably connected to the holder; and
   a collection container removably connected to the blood collector attachment, the container defining a collection cavity,
   wherein a collection container detachment member is provided between the blood collector attachment and the collection container to releasably attach the collection container to the blood collector attachment.
[Aspect 2] The device of aspect 1, wherein the collection container comprises a lid including a release tab that releases the collection container from the blood collector attachment when pressed.
[Aspect 3] The device of aspect 2, wherein the lid comprises an elastomer that acts as a spring pre-loading a locking feature to create resistance with the collection container.
[Aspect 4] The device of aspect 2, wherein the lid is connected to the collection container via a living hinge.
[Aspect 5] The device of aspect 4, wherein the living hinge has a reduced thickness or at least one cutout.
[Aspect 6] The device of aspect 2, wherein the blood collector attachment comprises a protrusion and the lid defines a recess to receive the protrusion to lock the collection container into the blood collector attachment.
[Aspect 7] The device of aspect 2, wherein the lid includes at least one guide tab to ensure a desired orientation of the lid on the collection container when closing the lid.
[Aspect 8] The device of aspect 1, wherein the collection container comprises an aligning protrusion extending from an outer surface of the collection container and the blood collector attachment defines an aligning slot on an inner surface of the blood collector attachment to orient the collection container in a desired position when inserted into the blood collector attachment.
[Aspect 9] The device of aspect 1, wherein a rotatable connection assembly is provided between the holder and the blood collector attachment.
[Aspect 10] The device of aspect 9, wherein, the rotatable connection assembly is configured to permit the collection container to rotate between a filling position and a stored position relative to the holder.
[Aspect 11] The device of aspect 9, wherein the rotatable connection assembly includes a socket and a post member that are held together via a friction fit.
[Aspect 12] The device of aspect 1, wherein the collection container detachment member provides haptic feedback when the collection container has been removed from the blood collector attachment.
[Aspect 13] A device for obtaining a blood sample, the device comprising:
   a blood collector attachment; and
   a collection container removably connected to the blood collector attachment, the container defining a collection cavity,
   wherein a collection container detachment member is provided between the blood collector attachment and the collection container to releasably attach the collection container to the blood collector attachment.
[Aspect 14] The device of aspect 13, wherein the collection container comprises a lid including a release tab that releases the collection container from the blood collector attachment when pressed.
[Aspect 15] The device of aspect 14, wherein the lid comprises an elastomer that acts as a spring pre-loading a locking feature to create resistance with the collection container.
[Aspect 16] The device of aspect 13, wherein the lid is connected to the collection container via a living hinge.
[Aspect 17] The device of aspect 13, wherein the blood collector attachment comprises a protrusion and the lid defines a recess to receive the protrusion to lock the collection container into the blood collector attachment.
[Aspect 18] The device of aspect 13, wherein the living hinge has a reduced thickness or at least one cutout.
[Aspect 19] The device of aspect 13, wherein the lid includes at least one guide tab to ensure a desired orientation of the lid on the collection container when closing the lid.
[Aspect 20] The device of aspect 13, wherein the collection container comprises an aligning protrusion extending from an outer surface of the collection container and the blood collector attachment defines an aligning slot on an inner surface of the blood collector attachment to orient the collection container in a desired position when inserted into the blood collector attachment.
[Aspect 21] The device of aspect 13, wherein the blood collector attachment defines a relief recess to provide a location for a user's hand to grip when using the device.
[Aspect 22] The device of aspect 13, wherein a recess is defined in the blood collector attachment above the release tab to receive a user's thumb when using the device.
[Aspect 23] The device of aspect 13, wherein, when the collection container is held in the blood collector attachment, the lid is held under pretension in the blood collector attachment.
[Aspect 24] The device of aspect 13, wherein the collection container detachment member provides haptic feedback when the collection container has been removed from the blood collector attachment.

## Claims

1. A device for obtaining a blood sample, the device comprising:
a blood collector attachment; and
a collection container removably connected to the blood collector attachment, the container defining a collection cavity,
wherein a collection container detachment member is provided between the blood collector attachment and the collection container to releasably attach the collection container to the blood collector attachment.

2. The device of claim 1, wherein the collection container comprises a lid including a release tab that releases the collection container from the blood collector attachment when pressed.

3. The device of claim 2, wherein the lid comprises an elastomer that acts as a spring pre-loading a locking feature to create resistance with the collection container.

4. The device of claim 1, wherein the lid is connected to the collection container via a living hinge.

5. The device of claim 1, wherein the blood collector attachment comprises a protrusion and the lid defines a recess to receive the protrusion to lock the collection container into the blood collector attachment.

6. The device of claim 1, wherein the living hinge has a reduced thickness or at least one cutout.

7. The device of claim 1, wherein the lid includes at least one guide tab to ensure a desired orientation of the lid on the collection container when closing the lid.

8. The device of claim 1, wherein the collection container comprises an aligning protrusion extending from an outer surface of the collection container and the blood collector attachment defines an aligning slot on an inner surface of the blood collector attachment to orient the collection container in a desired position when inserted into the blood collector attachment.

9. The device of claim 1, wherein the blood collector attachment defines a relief recess to provide a location for a user's hand to grip when using the device.

10. The device of claim 1, wherein a recess is defined in the blood collector attachment above the release tab to receive a user's thumb when using the device.

11. The device of claim 1, wherein, when the collection container is held in the blood collector attachment, the lid is held under pretension in the blood collector attachment.

12. The device of claim 1, wherein the collection container detachment member provides haptic feedback when the collection container has been removed from the blood collector attachment.
